# EUROPEAN PATENT SPECIFICATION

(11) **EP 4 310 130 B1**
(45) Date of publication and mention of the grant of the patent: **06.05.2026**
(21) Application number: 22771465.6
(22) Date of filing: 16.03.2022
(51) Int. Cl.: C08G 77/26, A61L 27/18, A61L 27/52, C07F 7/18, C08F 20/36, C08F 290/06, C08F 299/08

(54) **SILOXANE COMPOUND AND METHOD FOR PRODUCING SAME**
SILOXANVERBINDUNG UND VERFAHREN ZUR HERSTELLUNG DAVON
COMPOSÉ SILOXANE ET SON PROCÉDÉ DE PRODUCTION

(30) Priority: 17.03.2021 JP 2021043568
(43) Date of publication of application: 24.01.2024
(73) Proprietor: SHIN-ETSU CHEMICAL CO., LTD., Tokyo 1000005 (JP)
(72) Inventor: OKAMURA Kaoru, Annaka-shi, Gunma 379-0224 (JP)
(74) Representative: Ipsilon
(86) International application number: PCT/JP2022/011831
(87) International publication number: WO 2022/196720

(56) References cited:
- JP-A- 2014 153 710
- JP-A- 2016 501 925
- JP-A- 2017 517 585
- JP-A- 2019 210 363
- JP-A- S5 455 455
- US-A1- 2020 399 414
- US-B2- 9 329 306

## Description

### TECHNICAL FIELD

The present invention relates to a siloxane compound and a method for preparing the same. Specifically, the present invention provides a siloxane compound suitable for medical materials.

### BACKGROUND ART

Conventionally, monomers having siloxanes are known as a compound used for preparing medical materials including ophthalmic devices. For example, 3-[tris(trimethylsiloxy)silyl]propyl methacrylate (TRIS) is widely used as a monomer for medical materials. A polymer obtained by the copolymerization of TRIS with a hydrophilic monomer, such as 2-hydroxyethyl methacrylate, N,N-dimethylacrylamide, and N-vinyl-2-pyrrolidone, has beneficial characteristics such as high oxygen permeability. However, highly hydrophobic siloxane monomers do not have sufficient compatibility with such a hydrophilic monomer and, accordingly, in the preparation of a hydrogel prepared therefrom for medical materials, there is a problem in that phase separation occurs, causing the hydrogel to be cloudy. Therefore, attempts to improve compatibility have been performed by introducing a hydrophilic group in a siloxane monomer molecule.

Patent Literatures 1, 2, and 3 disclose a siloxane compound, which is methyl bis(trimethylsiloxy)silyl propyl glycerol methacrylate (SiGMA), having glycerol methacrylate represented by the following formula (a) or (a'). 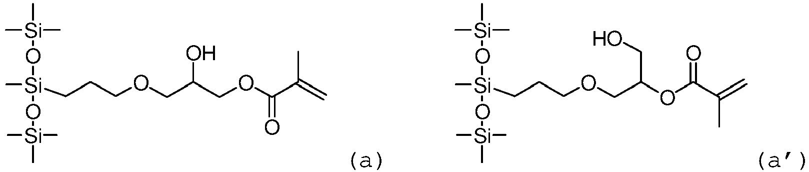 SiGMA is a monomer with a hydroxyl group in the molecule and formed by an addition reaction between methacrylic acid and an epoxy-modified siloxane compound, therefore it expresses satisfactory hydrophilicity. However, with SiGMA, a side reaction in an addition reaction of carboxylic acid with the epoxy-modified siloxane compound cannot be prevented, and there is a problem in that a purity of the intended siloxane compound decreases, thus SiGMA is not desirable as a raw material for medical materials.

Patent Literature 4 discloses a siloxane compound having a (meth)acrylamide group. Since the compound has an amide in the molecule, it expresses satisfactory hydrophilicity. Patent Literature 5 discloses self-assembled elastomers with molecularly encoded tissue-like softness, strain-adaptive stiffening and coloration. The disclosed copolymer blocks can be useful in, for example, the formation of polymer networks that replicate biological stress-strain behavior.

### PRIOR LITERATURES

Patent Literature 1: Japanese Patent Application Laid-Open No. Sho 54-55455(1979)
Patent Literature 2: Japanese Patent Application Laid-Open No. Sho 56-22325(1981)
Patent Literature 3: Japanese Patent Application Laid-Open No. 2004-182724
Patent Literature 4: Japanese Patent Application Laid-Open No. Hei 5-17577(1993)
Patent Literature 5: US 9,329,306 B2

### DISCLOSURE OF THE INVENTION

### PROBLEMS TO BE SOLVED BY THE INVENTION

However, most other monomers for medical materials that are polymerized with siloxane compounds have (meth)acrylates. The siloxane compound having a (meth)acrylamide group disclosed in Patent Literature 4 is inferior in terms of reactivity with monomer compounds having (meth)acrylates and causes phase separation during polymerization, which may make the obtained materials nonuniformity.

As described above, while a siloxane compound having a hydrophilic group has compatibility with hydrophilic monomers, it has problems with reactivity and purity. Therefore, the siloxane compound not desirable as a raw material of (meth)acrylic resins, particularly a raw material for medical materials. Consequently, providing a material having beneficial hydrophilicity, transparency, and strength is difficult with conventional siloxane compounds. Therefore, demand still exists for compounds and compositions that will overcome these drawbacks.

The present invention has been made in view of the above circumstances, and one of the purposes of the present invention is to provide a siloxane compound having an amide bond and a (meth)acrylate group. Further, other purpose of the present invention is to provide a method for preparing a siloxane compound having beneficial purity as a medical material.

### SOLUTIONS TO THE PROBLEMS

As a result of serious examination to solve the above-described problems, the present inventor found that a compound having a siloxane unit and a (meth)acrylate group, and having an amide bond at a linking group portion thereof, has excellent compatibility with other monomers including hydrophilic monomers, and further, has satisfactory reactivity. Further, the present inventor found that the siloxane compound obtained from a siloxane compound having an amino group, a lactone compound, and a (meth)acrylic acid halide has a high purity and, therefore, the siloxane compound is preferred as a raw material of a (meth)acrylic resin serving as medical materials.

That is, the present invention provides a siloxane compound represented by the following formula (1): 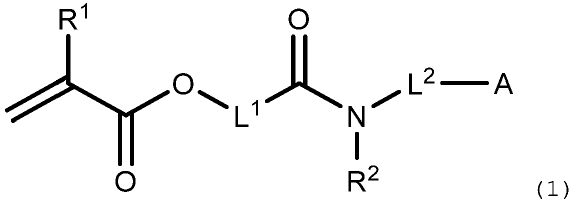 wherein R¹ is a hydrogen atom or a methyl group, R² is a hydrogen atom or a monovalent hydrocarbon group having 1 to 6 carbon atoms, L¹ is a divalent hydrocarbon group having 1 to 5 carbon atoms, L² is a divalent hydrocarbon group having 2 to 10 carbon atoms and which may contain an ether bond, and A is a polysiloxane unit represented by the following formula (2) or (3): 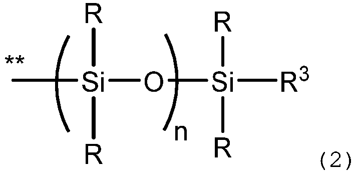 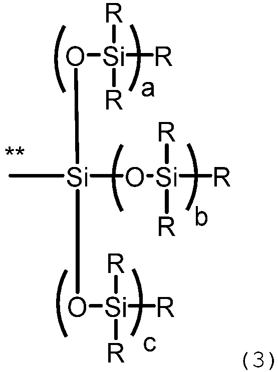 wherein n is an integer of 1 to 100, *a* is an integer of 0 to 10, *b* is an integer of 0 to 10, and c is an integer of 0 to 10, provided that a + *b* + c is 2 or more, R and R³ are, independently of each other, a monovalent hydrocarbon group having 1 to 10 carbon atoms, and the site marked with ** is a binding position to the group L² in formula (1).

Further, the present invention provides a method for preparing the siloxane compound, a polymer containing repeating units derived from the compound, and a medical material including the polymer, particularly an ophthalmic device.

### EFFECTS OF THE INVENTION

The siloxane compound of the present invention has excellent compatibility and reactivity with other monomers. Further, the siloxane compound obtained by the preparation method of the present invention has a high purity, and is therefore preferred as a raw material of a (meth)acrylic resin serving as a medical material.

### BRIEF DESCRIPTION OF THE DRAWING

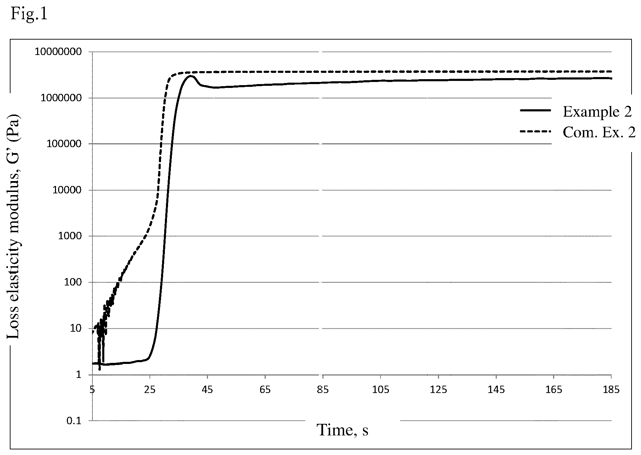

Fig. 1 is a graph having the results of measuring, by a rheometer, a viscoelastic change of a reaction solution in a polymerization reaction between a siloxane compound of Example 2 and other polymerizable monomers, and a viscoelastic change of a reaction solution in a polymerization reaction between a siloxane compound of Comparative Example 2 and other polymerizable monomers.

### DESCRIPTION OF PREFERRED EMBODIMENTS

The siloxane compound of the present invention will be described in detail below.

The siloxane compound of the present invention is a siloxane compound represented by the above formula (1). The siloxane compound is characterized by having a siloxane unit, an amide bond, and a (meth)acrylate group. The siloxane compound has an amide bond, so that it has excellent compatibility with other polymerizable monomers. The compound of the present invention has a (meth)acrylate group as the polymerizable group and, thereby, it has excellent reactivity with other (meth)acrylate monomers.

In the above formula (1), R¹ is a hydrogen atom or a methyl group. The methyl group is preferred.

In the above formula (1), R² is a hydrogen atom or a monovalent hydrocarbon group having 1 to 6 carbon atoms. Examples of the monovalent hydrocarbon group include an alkyl group such as a methyl group, an ethyl group, a propyl group, a butyl group, a pentyl group, and a hexyl group; a cycloalkyl group such as a cyclopentyl group and a cyclohexyl group; and an aryl group such as a phenyl group. R² is preferably a hydrogen atom or an alkyl group having 1 to 4 carbon atoms, and further preferably a hydrogen atom or a methyl group.

In the above formula (1), L¹ is a divalent hydrocarbon group having 1 to 5 carbon atoms. An aliphatic hydrocarbon group having 1 to 5 carbon atoms is preferred. Examples of L¹ include an alkylene group such as a methylene group, an ethylene group, a 1,3-propylene group, a 1-methyl propylene group, a 1,1-dimethyl propylene group, a 2-methyl propylene group, a 1,2-dimethyl propylene group, a 1,4-butylene group, a 2-methyl-1,4-butylene group, a 3-methyl-1,4-butylene group, and a 1,5-pentylene group. The 1,3-propylene group, the 1,4-butylene group, and the 1,5-pentylene group are preferred.

In the above formula (1), L² is a divalent hydrocarbon group having 2 to 10 carbon atoms, and which may contain an ether bond. An aliphatic hydrocarbon group having 2 to 10 carbon atoms is preferred, while 2 to 5 carbon atoms are more preferred. Examples of L² include an alkylene group such as an ethylene group, a 1,3-propylene group, a 1-methylpropylene group, a 1,1-dimethylpropylene group, a 2-methylpropylene group, a 1,2-dimethylpropylene group, a 1,1,2-trimethylpropylene group, a 1,4-butylene group, a 2-methyl-1,4-butylene group, a 2,2-dimethyl-1,4-butylene group, a 3-methyl-1,4-butylene group, a 2,3-dimethyl-1,4-butylene group, a 2,2,3-trimethyl-1,4-butylene group, a 1,5-pentylene group, a 1,6-hexanylene group, a 1,7-heptanylene group, a 1,8-octanylene group, a 1,9-nonanylene group, and a 1,10-decanylene group. Examples of the group containing an ether bond include polyoxyalkylene group such as a polyoxyethylene group, a polyoxypropylene group, and a polyoxyethylene-polyoxypropylene group. The 1,3-propylene group is preferred.

A is a polysiloxane unit represented by the following formula (2) or (3).

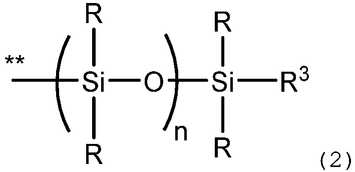

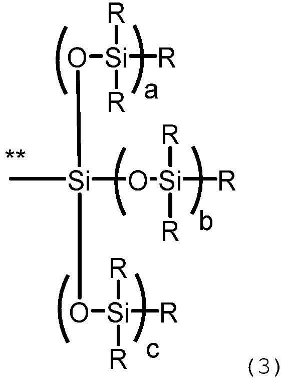

In formulae (2) and (3), *n* is an integer of 1 to 100, *a* is an integer of 0 to 10, *b* is an integer of 0 to 10, and c is an integer of 0 to 10, provided that *a + b + c* is 2 or more. Preferably, n is an integer of 2 to 20, *a* is 1, *b* is 1, and c is 1.

In formula (2) or (3), R and R³ are, independently of each other, a monovalent hydrocarbon group having 1 to 10 carbon atoms, and preferably 1 to 6 carbon atoms. Examples of R and R³ include an alkyl group such as a methyl group, an ethyl group, a propyl group, a butyl group, a pentyl group, a hexyl group, a heptyl group, an octyl group, a nonyl group, and a decyl group; a cycloalkyl group such as a cyclopentyl group and a cyclohexyl group; and an aryl group such as a phenyl group and a tolyl group. R and R³ are preferably an alkyl group having 1 to 10 carbon atoms, and more preferably 1 to 6 carbon atoms, or a phenyl group, and are preferably a methyl group or a butyl group. All of Rs in formula (3) is further preferably a methyl group. Further, in formula (2), R is preferably the methyl group, and R³ is preferably the butyl group. While the butyl group may be any of an n-butyl group, an s-butyl group, an isobutyl group and a t-butyl group, the n-butyl group is most preferred.

### [Method for preparing a siloxane compound]

A method for preparing the siloxane compound represented by formula (1) is described below.

The preparation method of the present invention includes an amidation step of reacting a siloxane compound having an amino group and represented by the following formula (5): 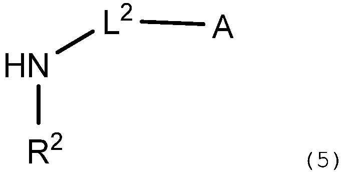
wherein R², L² and A are as described above,
with a lactone compound having 2 to 6 carbon atoms to obtain a hydroxy group-containing siloxane compound represented by the following formula (4): 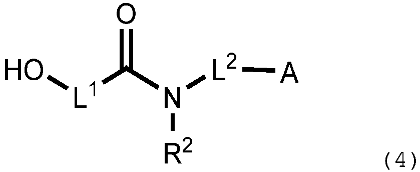
wherein R², L¹, L² and A are as described above,
and a (meth)acrylation step of reacting the hydroxy group-containing siloxane compound represented by formula (4) with a (meth)acrylic acid halide to obtain the siloxane compound represented by the above formula (1).

Each step is described in detail below.

### [Amidation Step]

The amidation step is a step of reacting the siloxane compound having an amino group and represented by the above formula (5) with a lactone compound to prepare a siloxane compound represented by the above formula (4).

Examples of the lactone compound having 2 to 6 carbon atoms include, but are not limited to, α-acetolactone, β-propiolactone, γ-butyrolactone, γ-valerolactone, δ-valerolactone, and ε-caprolactone. From the viewpoint of availability, γ-butyrolactone and ε-caprolactone are preferred.

The amidation step may be performed under reaction conditions in accordance with a conventionally-known method. For example, 1 mole or more of the lactone compound may be added to react with 1 mole of the siloxane compound having an amino group and represented by formula (5). The lactone compound is preferably 1.0 mole to 10.0 moles, further preferably 1.1 moles to 5.0 moles, and particularly preferably 1.2 moles to 3.0 moles, relative to 1 mole of the siloxane compound having an amino group. Adding 1 molar equivalent or more of the lactone compound to the siloxane compound having an amino group suppresses the presence of any remaining siloxane compound having an amino group, as well as side reactions. Further, while not limited, an upper limit value is preferably in the above range in terms of yield and economic efficiency.

While the amidation reaction may be performed either in air or in an inert gas atmosphere, the reaction is preferably performed under an inert gas atmosphere to suppress oxidation of the amino group. While a reaction temperature is not particularly limited, the reaction is preferably performed at a temperature of 0°C to 150°C, and more preferably in a range of 50°C to 120°C. The reaction may be performed in the presence of a solvent, a catalyst, or a stabilizer, and when using a solvent, the reaction temperature is preferably at a degree not exceeding a boiling point of the solvent used.

Examples of the solvent include a glycol ether-based solvent such as methyl cellosolve, ethyl cellosolve, isopropyl cellosolve, butyl cellosolve, propylene glycol monomethyl ether, diethylene glycol monomethyl ether, triethylene glycol monomethyl ether, polyethylene glycol monomethyl ether, ethylene glycol dimethyl ether, diethylene glycol dimethyl ether, triethylene glycol dimethyl ether, and polyethylene glycol dimethyl ether; an ester-based solvent such as ethyl acetate, butyl acetate, amyl acetate, ethyl lactate, and methyl benzoate; an aliphatic hydrocarbon-based solvent such as n-hexane, n-heptane, and n-octane; an alicyclic hydrocarbon-based solvent such as cyclohexane and ethylcyclohexane; a ketone-based solvent such as acetone, methyl ethyl ketone, and methyl isobutyl ketone; an aromatic hydrocarbon-based solvent such as benzene, toluene, and xylene; a halogenated hydrocarbon-based solvent such as dichloromethane, chloroform, 1,2-dichloroethane, carbon tetrachloride, chlorobenzene, and dichlorobenzene; a petroleum-based solvent such as industrial gasoline; an alcohol-based solvent such as methyl alcohol, ethyl alcohol, n-propyl alcohol, isopropyl alcohol, n-butyl alcohol, isobutyl alcohol, and tert-butyl alcohol; and a glycol-based solvent such as ethylene glycol, diethylene glycol, triethylene glycol, tetraethylene glycol, and polyethylene glycol. One kind of solvent may be used alone, or two or more kinds may be used in combination.

Examples of the catalyst include basic compounds, organophosphorus-based compounds, tertiary amines and Lewis acids. Examples of the basic compound include an alkali metal such as lithium hydroxide, sodium hydroxide, potassium hydroxide, calcium hydroxide, and magnesium hydroxide, and hydroxides of alkaline earth metals. Examples of the organophosphorus-based compound include tricyclohexylphosphine, tributylphosphine, trioctylphosphine, cyclohexyldiphenylphosphine, dicyclohexylphenylphosphine, butyldiphenylphosphine, dibutylphenylphosphine, octyldiphenylphosphine, dioctylphenylphosphine, and triphenylphosphine. Examples of the tertiary amine include trimethylamine, triethylamine, tripropylamine, tributylamine, diazabicycloundecene, diazabicyclononene, and 1-methylimidazole. Examples of the Lewis acid include boron trifluoride, aluminum chloride, methyldichloroaluminum, dimethylchloroaluminum, trimethylaluminum, magnesium chloride, magnesium bromide, titanium tetrachloride, dichlorotitanium bistriflate, biscyclopentadienyltitanium bistriflate, dichlorotitanium bisfluorosulfonate, tin tetrachloride, and tin(II) bistriflate. One kind of catalyst may be used alone, or two or more kinds may be used in combination.

Examples of the stabilizer include a phenol-based antioxidant, a phosphorus-based antioxidant, an amine-based antioxidant, and a sulfur-based antioxidant. Examples of the phenol-based antioxidant include, but are not particularly limited to, compounds selected from the group consisting of p-methoxyphenol, di-tert-butyl-p-cresol, pyrogallol, tert-butylcatechol, 4,4-thiobis(3-methyl-6-tert-butylphenol), 2,2'-methylenebis(4-methyl-6-t-butylphenol), phenolic resins, and cresol resins. Examples of the phosphorus-based antioxidant include, but are not particularly limited to, tris[2-[[2,4,8,10-tetrakis(1,1-dimethylethyl)dibenzo[d,f][1,3,2]dioxaphosphepin-6-yl]oxy]ethyl]amine, tris[2-[(4,6,9,11-tetra-tert-butyldibenzo[d,f][1,3,2]dioxaphosphepin-2-yl)oxy]ethyl]amine, and ethylbis(2,4-di-tert-butyl-6-methylphenyl) phosphite. Examples of the amine-based antioxidant include, but are not particularly limited to, tri or tetra C1-3 alkylpiperidine or a derivative thereof, bis(2,2,6,6-tetramethyl-4-piperidyl)oxalate, 1,2-bis(2,2,6,6-tetramethyl-4-piperidyloxy)ethane, phenylnaphthylamine, N,N'-diphenyl-1,4-phenylenediamine, and N-phenyl-N'-cyclohexyl-1,4-phenylenediamine. Examples of the sulfur-based antioxidant include, but are not particularly limited to, dilauryl thiodipropionate and distearyl thiodipropionate. One kind of stabilizer may be used alone, or two or more kinds may be used in combination.

In the above reaction, a reaction end point may be confirmed in accordance with a conventionally-known method by checking that a peak of a raw material compound has disappeared by, for example, thin-layer chromatography (TLC), high-performance liquid chromatography (HPLC), or gas chromatography (GC). After the reaction has ended, purification may be performed in accordance with a conventionally-known method. For example, a product can be isolated by washing an organic layer with water, then removing the solvent and the raw material. Further, reduced-pressure distillation or activated carbon processing may be used.

### [(Meth)Acrylation Step]

The (meth)acrylation step is a step of reacting the siloxane compound represented by the above formula (4) with a (meth)acrylic acid halide to prepare a compound represented by the above formula (1).

The (meth)acrylation step may be performed under reaction conditions in accordance with a conventionally-known method. For example, 1 mole or more of the (meth)acrylic acid halide may be added to react with 1 mole of the siloxane compound represented by formula (4), and the (meth)acrylic acid halide is preferably 1.0 mole to 3.0 moles, further preferably 1.1 moles to 2.0 moles, and particularly preferably 1.2 moles to 1.5 moles. Adding 1 molar equivalent or more of the (meth)acrylic acid halide to the siloxane compound suppreses the presence of any remaining siloxane compound. Further, while not limited, an upper limit value is preferably within the above range in terms of yield and economic efficiency.

While a reaction temperature is not particularly limited, the reaction is preferably performed at, for example, a temperature of 0°C to 80°C, and more preferably in a range of 0°C to 40°C. The reaction may be performed in the presence of a solvent, a catalyst, or a stabilizer. When using a solvent, the reaction temperature is preferably at a degree not exceeding a boiling point of the solvent used. The solvent, the catalyst, and the stabilizer may be ones that are conventionally known, and those exemplified for the amidation step may be used.

While the reaction may be performed either in air or in an inert gas atmosphere, the reaction is preferably performed under an inert gas atmosphere to suppress deactivation of the (meth)acrylic acid halide.

Examples of the (meth)acrylic acid halide include, but are not limited to, methacrylic acid chloride, acrylic acid chloride, methacrylic acid bromide, and acrylic acid bromide.

The (meth)acrylation reaction is preferably performed using a basic compound or a tertiary amine as a catalyst. Those exemplified as a catalyst for the above amidation step may be used as the basic compound or the tertiary amine, and one kind may be used alone, or two or more kinds may be used in combination.

While the solvents described in the above amidation step may be used as the solvent in the reaction, from among the alcohol-based solvents, the glycol-based solvents, and the glycol ether-based solvents, those having a hydroxyl group are not desirable. With solvents having a hydroxyl group, the hydroxyl group may react with the (meth)acrylic acid halide, which may lead to a poor reaction and a reduction in purity.

One example of the preparation method of the present invention is described below.

First, 1 molar equivalent of the siloxane compound represented by the above formula (5) and 2.0 molar equivalents of a lactone compound are heated and stirred at 100°C under a nitrogen atmosphere. The resulting mixture is reacted for about 6 hours, thereby completing the reaction. Next, the unreacted raw material is distilled off at a reduced pressure, thus making it possible to obtain a siloxane compound represented by the above formula (4).

Next, 1 molar equivalent of the siloxane compound represented by the above formula (4), 1.2 molar equivalents of triethylamine, and 2 mass equivalents of toluene are added, then 1.2 molar equivalents of (meth)acrylic acid chloride are added. After adding, the added components are stirred at room temperature. The resulting mixture is reacted for about 10 hours, thereby completing the reaction. At that time, the progress of the reaction may be confirmed by monitoring the siloxane compound or methacrylic acid chloride by GC measurement. After the reaction completes, the organic layer is washed with water, and the solvent and the unreacted raw material present in the organic layer are distilled off at a reduced pressure, thus making it possible to obtain a siloxane compound represented by the above formula (1).

In accordance with the above preparation method of the present invention, a high-purity siloxane compound which has few impurities such as by-products and is represented by the above formula (1) is obtained. That is, a high-purity siloxane compound, in which a proportion of the siloxane compound having one specific structure among the structures represented by formula (1) is 95 mass% or more, is obtained.

The siloxane compound of the present invention provides a polymer having repeating units derived from the addition polymerization of the (meth)acrylic group. The siloxane compound of the present invention has good compatibility with other compound(s) having a polymerizable group, such as a (meth)acrylic group (hereinafter referred to as polymerizable monomers), so that a colorless and transparent copolymer is obtained by copolymerizing the siloxane compound with polymerizable monomers. Further, the siloxane compound may be homopolymerized.

In the preparation of the copolymer containing repeating units derived from the siloxane compound and repeating units derived from the polymerization with other polymerizable monomer(s), a mass proportion of the repeating units derived from the siloxane compound may be 10 mass% or more, relative to the mass of the entire polymer. More specifically, the compound of the present invention may be copolymerized in an amount of preferably 10 parts by mass to 80 parts by mass, and more preferably 10 parts by mass to 60 parts by mass, relative to the total 100 parts by mass of the compound of the present invention and the other polymerizable monomer(s).

Examples of the polymerizable monomer include (meth)acrylic monomers other than siloxane compounds, acrylic acid derivatives, N-vinylpyrrolidone, other unsaturated aliphatic or aromatic compounds, and siloxane monomers having a polymerizable group, such as a (meth)acrylic group. More specific examples include a (meth)acrylic monomer such as (meth)acrylic acid, methyl(meth)acrylate, ethyl(meth)acrylate, (poly)ethylene glycol dimethacrylate, polyalkylene glycol mono(meth)acrylate, polyalkylene glycol monoalkyl ether (meth)acrylate, trifluoroethyl (meth)acrylate, 2-hydroxyethyl (meth)acrylate, and 2,3-dihydroxypropyl (meth)acrylate; an acrylic acid derivative such as N,N-dimethylacrylamide, N,N-diethylacrylamide, N-acryloylmorpholine, and N-methyl (meth)acrylamide; N-vinylpyrrolidone and other unsaturated aliphatic or aromatic compounds such as crotonic acid, cinnamic acid, and vinylbenzoic acid; and a siloxane monomer having a polymerizable group such as a (meth)acrylic group, other than that of the above formula (1). One kind may be used alone, or two or more kinds may be used in combination.

From the aspect of reactivity with the siloxane compound of the present invention, at least one of the other polymerizable monomers is preferably a (meth)acrylic monomer other than a siloxane compound. The siloxane compound of the present invention and the (meth)acrylic monomer other than a siloxane compound may be polymerized in an amount of 50 parts by mass or more, preferably 50 parts by mass to 100 parts by mass, and more preferably 70 parts by mass to 100 parts by mass, relative to the total 100 parts by mass of the siloxane compound of the present invention and the other polymerizable monomers. That is, in the obtained copolymer, the mass proportion of the repeating units derived from the siloxane compound of the present invention and the repeating units derived from the (meth)acrylic monomer may be 50 mass% or more, and preferably 70 mass% or more, relative to the total mass of the copolymer. While an upper limit is not particularly limited, the repeating units derived from the siloxane compound of the present invention and the repeating units derived from the (meth)acrylic monomer preferably account for 100 mass%.

The copolymerization of the siloxane compound with the other polymerizable monomers above may be performed by a conventionally-known method. For example, the copolymerization can be performed using a known polymerization initiator, such as a heat polymerization initiator or a photopolymerization initiator. Examples of the polymerization initiator include 2-hydroxy-2-methyl-1-phenyl-propan-1-one, azobisisobutyronitrile, azobisdimethylvaleronitrile, benzoyl peroxide, tert-butyl hydroperoxide, cumene hydroperoxide, and 2,2'-azobis(2-methylpropionamidine) dihydrochloride. One kind of these polymerization initiators may be used alone, or two or more kinds may be used in combination. A blend amount of the polymerization initiator may be 0.001 part by mass to 2 parts by mass, and preferably 0.01 part by mass to 1 part by mass, relative to the total 100 parts by mass of the components to be polymerized.

The polymer containing the repeating units derived from the siloxane compound has excellent hydrophilicity. Further, a hydrogel obtained from the polymer has excellent transparency and strength. Consequently, the compound of the present invention is suited for the preparation of medical materials, such as ophthalmic devices, contact lenses, intraocular lenses, and artificial corneas. The method for preparing a medical material using the polymer is not particularly limited and may be performed in accordance with a conventionally-known preparation method for medical materials. For example, a cutting process or a casting (molding) process may be used when forming into the shape of a lens, such as a contact lens or an intraocular lens.

### EXAMPLES

The present invention will be explained below in further detail with reference to a series of the Examples and the Comparative Examples, though the present invention is in no way limited by these Examples. In the following Examples, ¹H-NMR analysis was conducted with an ECS400 manufactured by JEOL using deuterated chloroform as a measurement solvent.

All gas chromatography (GC) measurements were performed under the following conditions.
An Agilent 7890B Gas Chromatography system (FID detector) was used.
Capillary column: HP-5MS (0.25 mm × 30 m × 0.25 µm) manufactured by J&W
Heating program: 50°C (5 minutes) → 10°C/min → 250°C (held) Temperature at injection port: 250°C
FID detector temperature: 300°C
Carrier gas: helium (1.0 ml/min)
Split ratio: 50:1, and Injected volume: 1 µL

Further, all rheometer measurements were performed under the following conditions.
Performed using an OMNICURE series 2000 manufactured by Excelitas Technologies connected with a TA Instruments
DISCOVERY HR-2.
UV irradiation intensity: 45 mW/cm²
Geometry: 20 mm quartz plate
Temperature: 25°C
Environment: Under N₂ atmosphere
Period: 10 minutes
Gap: 200 µm
Sampling: Oscillation-Fast Sampling
Sample amount: 100 µL

### Synthesis Example 1

To a 300-mL three-necked eggplant flask equipped with a Dimroth condenser and a thermometer, were added 80.0 g of 3-aminopropyltris(trimethylsiloxy)silane and 70.0 g of γ-butyrolactone under a nitrogen atmosphere and, then, heated to 100°C and aged for 6 hours. After reacting, the unreacted raw material were distilled off at a reduced pressure and at an inside temperature of 120°C to obtain a colorless and transparent liquid represented by the following formula (8B). The yield was 97.0 g.

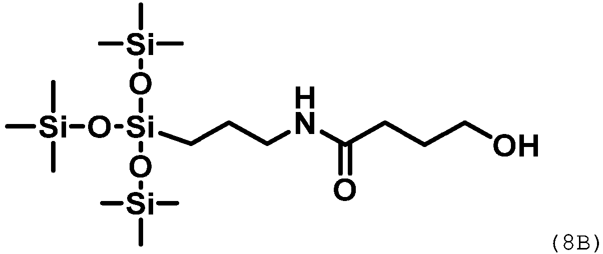

### Synthesis Example 2

Synthesis Example 1 was repeated, except that 3-aminopropyltris(trimethylsiloxy)silane in Synthesis Example 1 was replaced with a siloxane compound represented by the following formula (9A), to obtain a colorless and transparent liquid represented by the following formula (9B). The yield was 96.9 g.

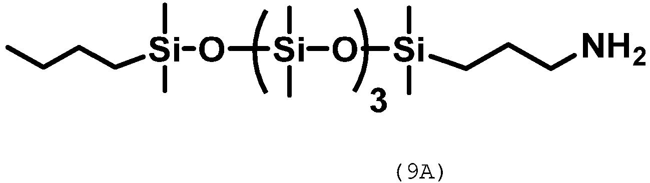

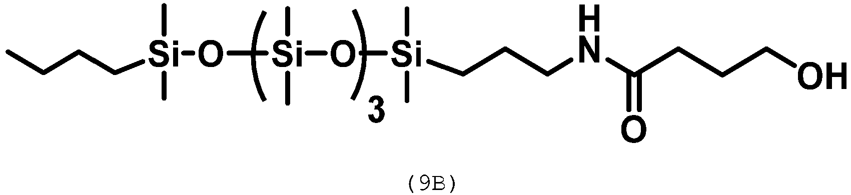

### Synthesis Example 3

Synthesis Example 1 was repeated, except that 3-aminopropyltris(trimethylsiloxy)silane in Synthesis Example 1 was replaced with a siloxane compound represented by the following formula (10A), to obtain a colorless and transparent liquid represented by the following formula (10B). The yield was 91.2 g.

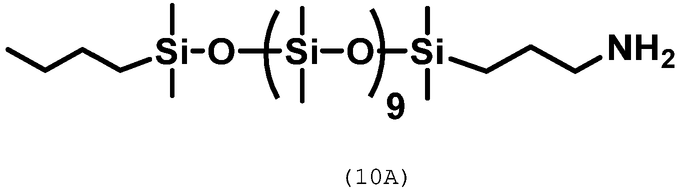

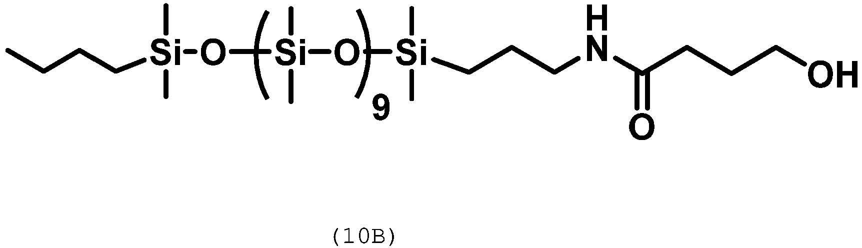

### Synthesis Example 4

Synthesis Example 1 was repeated, except that 3-aminopropyltris(trimethylsiloxy)silane in Synthesis Example 1 was replaced with a siloxane compound represented by the following formula (11A), to obtain a colorless and transparent liquid represented by the following formula (11B). The yield was 93.6 g.

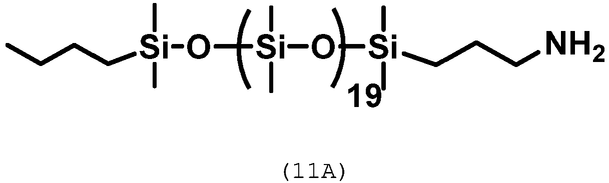

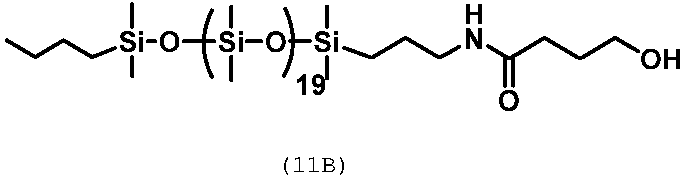

### Synthesis Example 5

Synthesis Example 2 was repeated, except that γ-butyrolactone in Synthesis Example 2 was replaced with ε-caprolactone, to obtain a colorless and transparent liquid represented by the following formula (12B). The yield was 98.1 g.

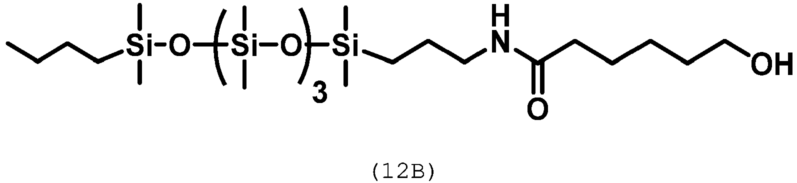

### Example 1

To a 500-mL three-necked eggplant flask equipped with a Dimroth condenser and a thermometer, were added 80.0 g of the siloxane compound (8B) obtained in Synthesis Example 1, 22.2 g of triethylamine, and 240.0 g of toluene were added under a nitrogen atmosphere, and cooled to 10°C. 22.8 g of methacrylic acid chloride was added dropwise and aged at 20°C for 10 hours. After reacting, the resulting mixture was washed with deionized water three times, and the solvent and the unreacted raw material were distilled off at a reduced pressure and at an inside temperature of 80°C to obtain a colorless and transparent liquid represented by the following formula (8C). The yield was 91.0 g. The GC purity was 98.8%. The ¹H-NMR data is shown below. 0.0ppm(27H), 0.4ppm(2H), 1.6ppm(2H), 1.9ppm(3H), 2.0ppm(2H), 2.3ppm(2H), 3.2ppm(2H), 4.2ppm(2H), 5.6ppm(1H), 6.1ppm(1H)

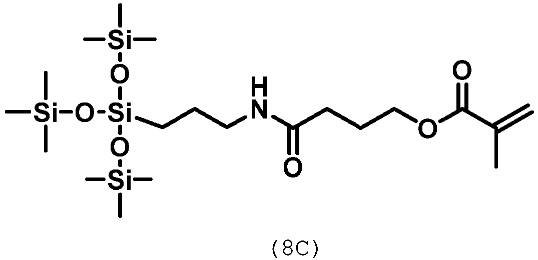

### Example 2

Example 1 was repeated, except that the siloxane compound (8B) in Example 1 was replaced with the siloxane compound represented by the above formula (9B), to obtain a colorless and transparent liquid represented by the following formula (9C). The yield was 86.6 g. The GC purity was 98.4%. The ¹H-NMR data is shown below. 0.0ppm(30H), 0.5ppm(4H), 0.9ppm(3H), 1.3ppm(4H), 1.6ppm(2H), 1.9ppm(3H), 2.0ppm(2H), 2.3ppm(2H), 3.2ppm(2H), 4.2ppm(2H), 5.6ppm(1H), 6.1ppm(1H)

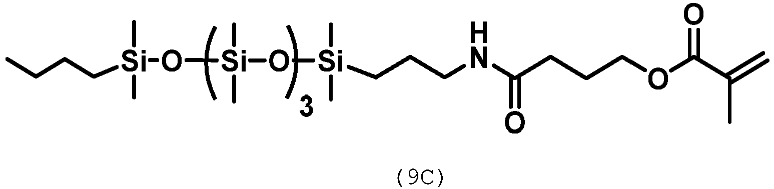

### Example 3

Example 1 was repeated, except that the siloxane compound (8B) in Example 1 was replaced with the siloxane compound represented by the above formula (10B), to obtain a colorless and transparent liquid represented by the following formula (10C). The yield was 83.7 g. The GC purity was 98.0%. The ¹H-NMR data is shown below. 0.0ppm(66H), 0.5ppm(4H), 0.9ppm(3H), 1.3ppm(4H), 1.6ppm(2H), 1.9ppm(3H), 2.0ppm(2H), 2.3ppm(2H), 3.2ppm(2H), 4.2ppm(2H), 5.6ppm(1H) and 6.1ppm(1H)

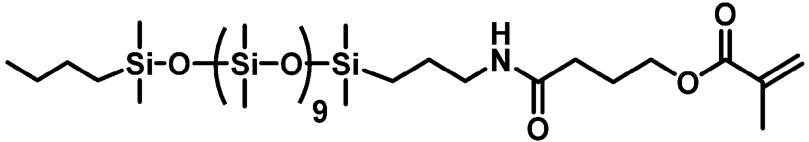

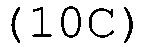

### Example 4

Example 1 was repeated, except that the siloxane compound (8B) in Example 1 was replaced with the siloxane compound represented by the above formula (11B), to obtain a colorless and transparent liquid represented by the following formula (11C). The yield was 84.4 g. The ¹H-NMR data is shown below.

0.0ppm(126H), 0.5ppm(4H), 0.9ppm(3H), 1.3ppm(4H), 1.6ppm(2H), 1.9ppm(3H), 2.0ppm(2H), 2.3ppm(2H), 3.2ppm(2H), 4.2ppm(2H), 5.6ppm(1H) and 6.1ppm(1H)

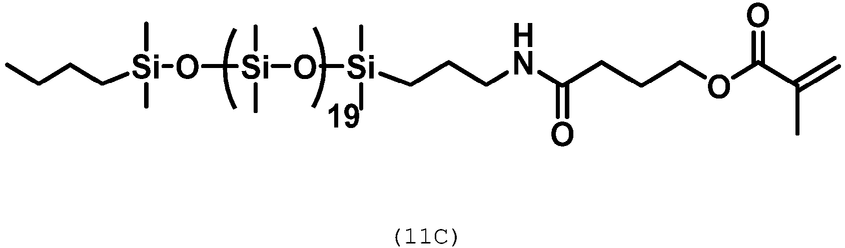

### Example 5

Example 1 was repeated, except that the siloxane compound (8B) in Example 1 was replaced with the siloxane compound represented by the above formula (12B), to obtain a colorless and transparent liquid represented by the following formula (12C). The yield was 89.4 g. The GC purity was 98.2%. The ¹H-NMR data is shown below. 0.0ppm(30H), 0.5ppm(4H), 0.9ppm(3H), 1.3ppm(8H), 1.6ppm(2H), 1.9ppm(3H), 2.0ppm(2H), 2.3ppm(2H), 3.2ppm(2H), 4.2ppm(2H), 5.6ppm(1H), 6.1ppm(1H)

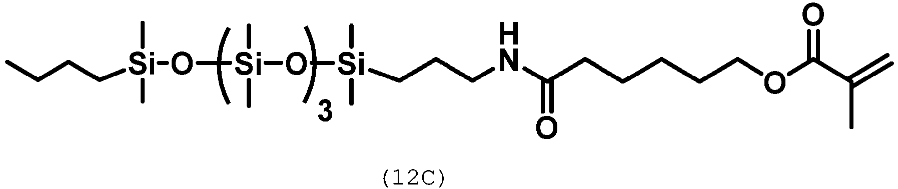

Other polymerizable monomer compounds used in the Examples and Comparative Examples are as follows:
HEMA: 2-hydroxyethyl methacrylate
MMA: methyl methacrylate

In Comparative Examples 1 and 2, the following siloxane compounds were used instead of the siloxane compound of the present invention.
TRIS: 3-[tris(trimethylsiloxy)silyl]propyl methacrylate 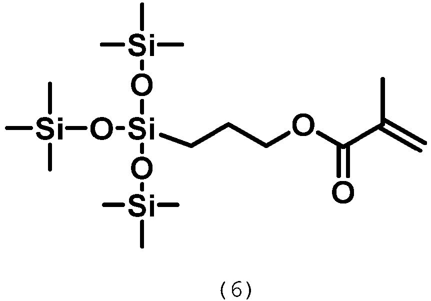
AM-PDMS: monoacrylamide polydimethylsiloxane (number average molecular weight 500)

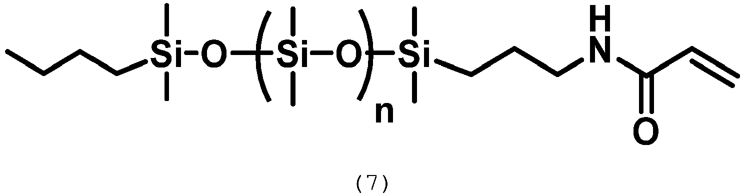

Each of the siloxane compounds obtained in the above Examples, TRIS, or AM-PDMS were mixed with HEMA and MMA, which are the other polymerizable monomers, and Irgacure 1173 (Irg1173), which is the polymerization initiator, in a blend ratio shown in Table 1 below and stirred for 10 minutes. After stirring, the mixtures were left to stand for 10 minutes, and the state of the solutions were visually observed. The evaluation results based on the following criteria are shown in Table 1.

### [Compatibility]

A: Uniform and transparent
B: Non-uniform or cloudy

### [Viscoelastic Change]

After the above evaluation of compatibility, the solutions were sufficiently degassed by bubbling with nitrogen gas for 5 minutes, then the change in viscoelasticity of the obtained solutions was measured with the rheometer. The measuring conditions of the rheometer are as described above. Fig. 1 shows the rheometer measurement results of the polymerization reaction using the siloxane compound of Example 2 and the polymerization reaction using the siloxane compound of Comparative Example 2.

**[Table 1]**

| Component, Part by mass | | Ex.1 | Ex.2 | Ex.3 | Ex.4 | Ex.5 | Com. Ex.1 | Com. Ex.2 |
|---|---|---|---|---|---|---|---|---|
| Siloxane compound | 8C | 50 | 0 | 0 | 0 | 0 | 0 | 0 |
| | 9C | 0 | 50 | 0 | 0 | 0 | 0 | 0 |
| | 10C | 0 | 0 | 50 | 0 | 0 | 0 | 0 |
| | 11C | 0 | 0 | 0 | 50 | 0 | 0 | 0 |
| | 12C | 0 | 0 | 0 | 0 | 50 | 0 | 0 |
| Comparative compound | TRIS | 0 | 0 | 0 | 0 | 0 | 50 | 0 |
| | AM-PDMS | 0 | 0 | 0 | 0 | 0 | 0 | 50 |
| Acryl monomer | HEMA | 40 | 40 | 40 | 40 | 40 | 40 | 40 |
| | MMA | 9 | 9 | 9 | 9 | 9 | 9 | 9 |
| Polymerization initiator | IRG1173 | 1 | 1 | 1 | 1 | 1 | 1 | 1 |
| Result | Compatibility | A | A | A | A | A | B | A |

As shown in Table 1, the compound of the present invention has an amide and a (meth)acrylate and, thereby, the compound has excellent compatibility with other polymerizable monomers including hydrophilic monomers, compared to conventional siloxane monomers.

Further, as shown in Fig. 1, the siloxane compound of the present invention exhibits one-phase polymerization behavior and is said to be uniformly polymerized with other (meth)acrylic monomers. In contrast, AM-PDMS having an acrylamide has good compatibility with other polymerizable monomers, but AM-PDMS exhibits two-phase polymerization behavior in which an inclination changes in a graph of the elastic modulus as shown in Fig. 1 and, thus, AM-PDMS is not uniformly polymerized with other (meth)acrylic monomers.

As described above, since the siloxane compound of the present invention has excellent compatibility and reactivity with other polymerizable monomers and, further, the siloxane compound has a high purity. Therefore, the siloxane compound provides a (meth)acrylic resin having beneficial transparency and strength.

### INDUSTRIAL APPLICABILITY

The compound of the present invention has excellent compatibility and reactivity with (meth)acrylic monomer(s). The compound of the present invention is beneficial as a monomer in the preparation of medical materials such as, for example, ophthalmic devices, contact lenses, intraocular lenses, artificial corneas and spectacle lenses.

## Claims

1. A siloxane compound represented by the following formula (1): 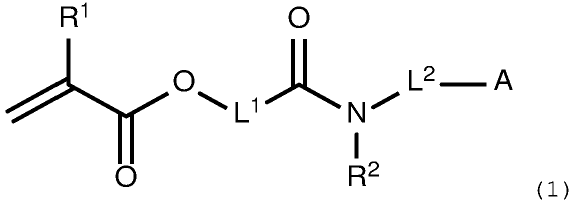
wherein R¹ is a hydrogen atom or a methyl group, R² is a hydrogen atom or a monovalent hydrocarbon group having 1 to 6 carbon atoms, L¹ is a divalent hydrocarbon group having 1 to 5 carbon atoms, L² is a divalent hydrocarbon group having 2 to 10 carbon atoms and which may contain an ether bond, and A is a polysiloxane unit represented by the following formula (2) or (3): 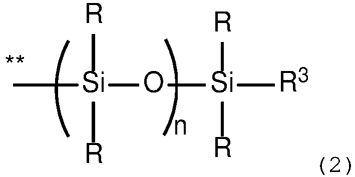 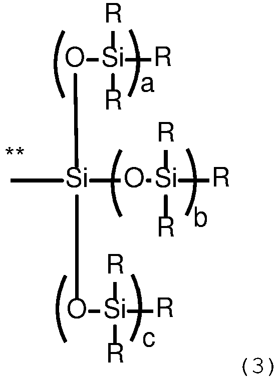
wherein n is an integer of 1 to 100, *a* is an integer of 0 to 10, *b* is an integer of 0 to 10, and c is an integer of 0 to 10, provided that *a + b + c* is 2 or more, R and R³ are, independently of each other, a monovalent hydrocarbon group having 1 to 10 carbon atoms, and the site marked with ** is a binding position to the group L² in formula (1).

2. The siloxane compound according to claim 1, wherein R¹ is a methyl group.

3. The siloxane compound according to claim 1 or 2, wherein A is represented by said formula (2) and n is an integer of 2 to 20.

4. The siloxane compound according to claim 1 or 2, wherein A is represented by said formula (3) and a is 1, *b* is 1 and c is 1.

5. The siloxane compound according to any one of claims 1 to 4, wherein R and R³ in said formulae (2) and (3) are, independently of each other, an alkyl group having 1 to 6 carbon atoms, or a phenyl group.

6. The siloxane compound according to claim 5, wherein R in said formula (2) is a methyl group and R³ in said formula (2) is a butyl group.

7. The siloxane compound according to claim 5, wherein R in said formula (3) is a methyl group.

8. The siloxane compound according to any one of claims 1 to 7, wherein a proportion of the siloxane compound having one specific structure among the structures represented by formula (1) is 95 mass% or more.

9. A polymer derived by polymerization of the siloxane compound according to any one of claims 1 to 8.

10. A copolymer derived by polymerization of the siloxane compound according to any one of claims 1 to 8 with other polymerizable monomer(s).

11. The copolymer according to claim 10, wherein a mass proportion of the repeating units derived from the siloxane compound is 10 mass% or more to 80 mass% or less, relative to the mass of the copolymer.

12. The copolymer according to claim 10 or 11, wherein at least one of the other polymerizable monomer(s) is a (meth)acrylic monomer other than a siloxane compound, and a total mass proportion of the repeating units derived from the siloxane compound and the repeating units derived from the (meth)acrylic monomer is 50 mass% or more, relative to the mass of the copolymer.

13. A hydrogel comprising the polymer according to claim 9 or the copolymer according to any one of claims 10 to 12.

14. A medical material comprising the polymer according to claim 9 or the copolymer according to any one of claims 10 to 12.

15. A method for preparing the siloxane compound represented by the following formula (1): 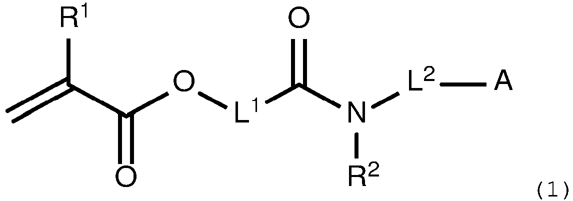
wherein R¹ is a hydrogen atom or a methyl group, R² is a hydrogen atom or a monovalent hydrocarbon group having 1 to 6 carbon atoms, L¹ is a divalent hydrocarbon group having 1 to 5 carbon atoms, L² is a divalent hydrocarbon group having 2 to 10 carbon atoms and which may contain an ether bond, and A is a polysiloxane unit represented by the following formula (2) or (3): 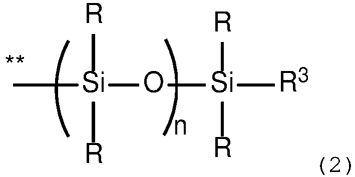 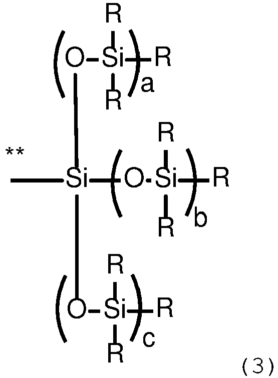
wherein n is an integer of 1 to 100, *a* is an integer of 0 to 10, *b* is an integer of 0 to 10, and c is an integer of 0 to 10, provided that *a + b + c* is 2 or more, R and R³ are, independently of each other, a monovalent hydrocarbon group having 1 to 10 carbon atoms, and the site marked with ** is a binding position to the group L² in formula (1),
wherein the method comprises a step of reacting a siloxane compound having an amino group and represented by the following formula (5): 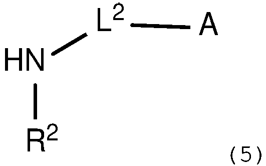
wherein R², L² and A are as described above,
with a lactone compound having 2 to 6 carbon atoms to obtain a siloxane compound represented by the following formula (4): 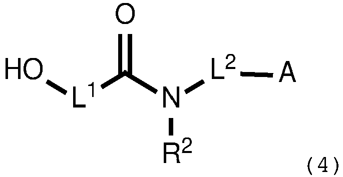
wherein R², L¹, L² and A are as described above,
and a step of reacting the siloxane compound represented by said formula (4) with a (meth)acrylic acid halide to obtain the compound represented by said formula (1).

16. The method according to claim 15, wherein the lactone compound is selected from the group consisting of α-acetolactone, β-propiolactone, γ-butyrolactone, γ-valerolactone, δ-valerolactone and ε-caprolactone.

17. The method according to claim 15 or 16, wherein the (meth)acrylic acid halide is methacrylic acid chloride, acrylic acid chloride, methacrylic acid bromide or acrylic acid bromide.

18. The method according to any one of claims 15 to 17, wherein a proportion of the siloxane compound having one specific structure among the structures represented by said formula (1) is 95 mass% or more.

## Patentansprüche

1. Siloxanverbindung, dargestellt durch die folgende Formel (1) : 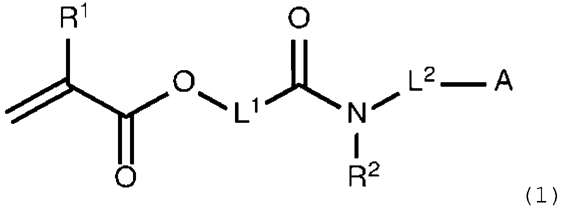
wobei R¹ ein Wasserstoffatom oder eine Methylgruppe ist, R² ein Wasserstoffatom oder eine einwertige Kohlenwasserstoffgruppe mit 1 bis 6 Kohlenstoffatomen ist, L¹ eine zweiwertige Kohlenwasserstoffgruppe mit 1 bis 5 Kohlenstoffatomen ist, L² eine zweiwertige Kohlenwasserstoffgruppe mit 2 bis 10 Kohlenstoffatomen ist, die eine Etherbindung enthalten kann, und A eine Polysiloxaneinheit dargestellt durch die folgende Formel (2) oder (3) ist: 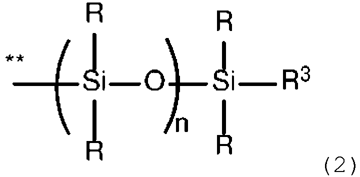 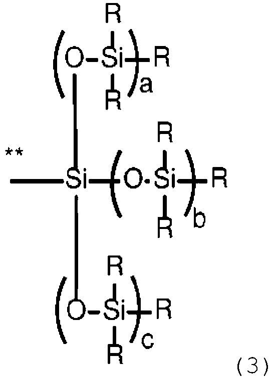
wobei n eine ganze Zahl von 1 bis 100 ist, a eine ganze Zahl von 0 bis 10 ist, b eine ganze Zahl von 0 bis 10 ist und c eine ganze Zahl von 0 bis 10 ist, mit der Maßgabe, dass a + b + c 2 oder mehr ist, R und R³ unabhängig voneinander eine einwertige Kohlenwasserstoffgruppe mit 1 bis 10 Kohlenstoffatomen sind und die mit ** gekennzeichnete Stelle eine Bindungsposition an die Gruppe L² in Formel (1) ist.

2. Siloxanverbindung nach Anspruch 1, wobei R¹ eine Methylgruppe ist.

3. Siloxanverbindung nach Anspruch 1 oder 2, wobei A durch die Formel (2) dargestellt wird und n eine ganze Zahl von 2 bis 20 ist.

4. Siloxanverbindung nach Anspruch 1 oder 2, wobei A durch die Formel (3) dargestellt wird und a 1 ist, b 1 ist und c 1 ist.

5. Siloxanverbindung nach einem der Ansprüche 1 bis 4, wobei R und R³ in den Formeln (2) und (3) unabhängig voneinander eine Alkylgruppe mit 1 bis 6 Kohlenstoffatomen oder eine Phenylgruppe sind.

6. Siloxanverbindung nach Anspruch 5, wobei R in der Formel (2) eine Methylgruppe ist und R³ in der Formel (2) eine Butylgruppe ist.

7. Siloxanverbindung nach Anspruch 5, wobei R in der Formel (3) eine Methylgruppe ist.

8. Siloxanverbindung nach einem der Ansprüche 1 bis 7, wobei ein Anteil der Siloxanverbindung mit einer spezifischen Struktur unter den durch Formel (1) dargestellten Strukturen 95 Masse-% oder mehr beträgt.

9. Polymer, abgeleitet durch Polymerisation der Siloxanverbindung nach einem der Ansprüche 1 bis 8.

10. Copolymer, abgeleitet durch Polymerisation der Siloxanverbindung nach einem der Ansprüche 1 bis 8 mit anderen polymerisierbaren Monomerr(en).

11. Copolymer nach Anspruch 10, wobei ein Masseanteil der von der Siloxanverbindung abgeleiteten Wiederholungseinheiten 10 Masse-% oder mehr bis 80 Masse-% oder weniger, bezogen auf die Masse des Copolymers, beträgt.

12. Copolymer nach Anspruch 10 oder 11, wobei wenigstens eines der anderen polymerisierbaren Monomere ein von einer Siloxanverbindung verschiedenes (Meth)acrylmonomer ist und ein Gesamt-Masseanteil der von der Siloxanverbindung abgeleiteten Wiederholungseinheiten und der von dem (Meth)acrylmonomer abgeleiteten Wiederholungseinheiten 50 Masse-% oder mehr, bezogen auf die Masse des Copolymers, beträgt.

13. Hydrogel umfassend das Polymer nach Anspruch 9 oder das Copolymer nach einem der Ansprüche 10 bis 12.

14. Medizinisches Material, umfassend das Polymer nach Anspruch 9 oder das Copolymer nach einem der Ansprüche 10 bis 12.

15. Verfahren zur Herstellung der Siloxanverbindung dargestellt durch die folgende Formel (1): 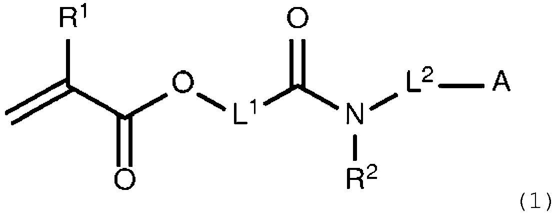
wobei R¹ ein Wasserstoffatom oder eine Methylgruppe ist, R² ein Wasserstoffatom oder eine einwertige Kohlenwasserstoffgruppe mit 1 bis 6 Kohlenstoffatomen ist, L¹ eine zweiwertige Kohlenwasserstoffgruppe mit 1 bis 5 Kohlenstoffatomen ist, L² eine zweiwertige Kohlenwasserstoffgruppe mit 2 bis 10 Kohlenstoffatomen ist, die eine Etherbindung enthalten kann, und A eine Polysiloxaneinheit dargestellt durch die folgende Formel (2) oder (3) ist: 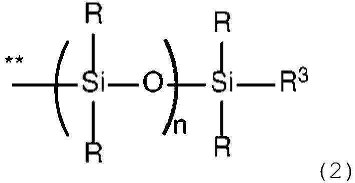 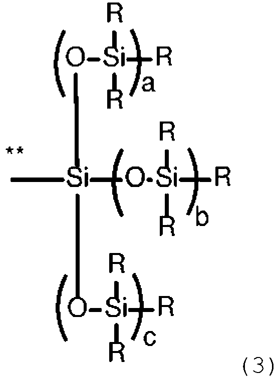
wobei n eine ganze Zahl von 1 bis 100 ist, a eine ganze Zahl von 0 bis 10 ist, b eine ganze Zahl von 0 bis 10 ist und c eine ganze Zahl von 0 bis 10 ist, mit der Maßgabe, dass a + b + c 2 oder mehr ist, R und R³ unabhängig voneinander eine einwertige Kohlenwasserstoffgruppe mit 1 bis 10 Kohlenstoffatomen sind und die mit ** gekennzeichnete Stelle eine Bindungsposition an die Gruppe L² in Formel (1) ist,
wobei das Verfahren einen Schritt des Umsetzens einer Siloxanverbindung mit einer Aminogruppe und dargestellt durch die folgende Formel (5): 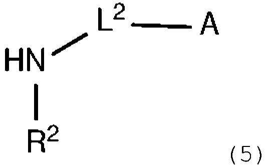
wobei R², L² und A wie oben beschrieben sind,
mit einer Lactonverbindung mit 2 bis 6 Kohlenstoffatomen, um eine Siloxanverbindung dargestellt durch die folgende Formel (4) zu erhalten: 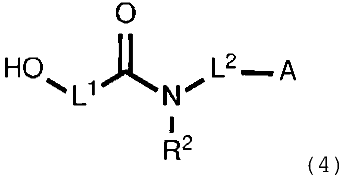
wobei R², L¹, L² und A wie oben beschrieben sind,
und einen Schritt des Umsetzens der durch die Formel (4) dargestellten Siloxanverbindung mit einem (Meth)acrylsäurehalogenid, um die durch die Formel (1) dargestellte Verbindung zu erhalten, umfasst.

16. Verfahren nach Anspruch 15, wobei die Lactonverbindung ausgewählt ist aus der Gruppe bestehend aus α-Acetolacton, β-Propiolacton, γ-Butyrolacton, γ-Valerolacton, δ-Valerolacton und ε-Caprolacton.

17. Verfahren nach Anspruch 15 oder 16, wobei das (Meth)acrylsäurehalogenid Methacrylsäurechlorid, Acrylsäurechlorid, Methacrylsäurebromid oder Acrylsäurebromid ist.

18. Verfahren nach einem der Ansprüche 15 bis 17, wobei ein Anteil der Siloxanverbindung mit einer spezifischen Struktur unter den durch die Formel (1) dargestellten Strukturen 95 Masse-% oder mehr beträgt.

## Revendications

1. Composé siloxane représenté par la formule (1) suivante : 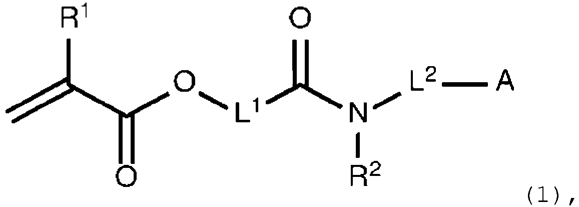
dans laquelle R¹ est un atome d'hydrogène ou un groupe méthyle, R² est un atome d'hydrogène ou un groupe hydrocarboné monovalent ayant de 1 à 6 atomes de carbone, L¹ est un groupe hydrocarboné divalent ayant de 1 à 5 atomes de carbone, L² est un groupe hydrocarboné divalent ayant de 2 à 10 atomes de carbone et pouvant contenir une liaison éther, et A est un motif polysiloxane représenté par la formule suivante (2) ou (3) : 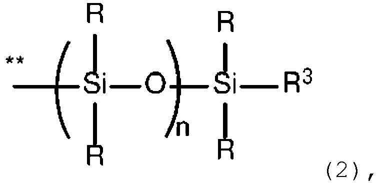 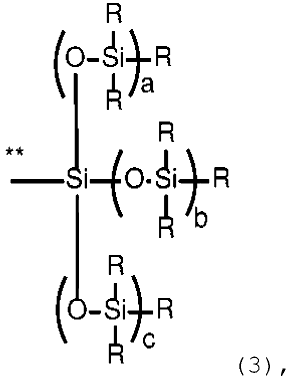
dans laquelle n est un entier de 1 à 100, a est un entier de 0 à 10, b est un entier de 0 à 10, et c est un entier de 0 à 10, à condition que a + b + c soit 2 ou plus, R et R³ sont, indépendamment l'un de l'autre, un groupe hydrocarboné monovalent ayant 1 à 10 atomes de carbone, et le site marqué par ** est une position de liaison au groupe L² dans la formule (1).

2. Composé siloxane selon la revendication 1, dans lequel R¹ est un groupe méthyle.

3. Composé siloxane selon la revendication 1 ou 2, dans lequel A est représenté par ladite formule (2) et n est un entier de 2 à 20.

4. Composé siloxane selon la revendication 1 ou 2, dans lequel A est représenté par ladite formule (3) et a est 1, b est 1 et c est 1.

5. Composé siloxane selon l'une quelconque des revendications 1 à 4, dans lequel R et R³ dans lesdites formules (2) et (3) sont, indépendamment l'un de l'autre, un groupe alkyle ayant 1 à 6 atomes de carbone, ou un groupe phényle.

6. Composé siloxane selon la revendication 5, dans lequel R dans ladite formule (2) est un groupe méthyle et R³ dans ladite formule (2) est un groupe butyle.

7. Composé siloxane selon la revendication 5, dans lequel R dans ladite formule (3) est un groupe méthyle.

8. Composé siloxane selon l'une quelconque des revendications 1 à 7, dans lequel une proportion du composé siloxane ayant une structure spécifique parmi les structures représentées par la formule (1) est de 95 % en masse ou plus.

9. Polymère dérivé par polymérisation du composé siloxane selon l'une quelconque des revendications 1 à 8.

10. Copolymère dérivé par polymérisation du composé siloxane selon l'une quelconque des revendications 1 à 8 avec un ou plusieurs autres monomères polymérisables.

11. Copolymère selon la revendication 10, dans lequel une proportion en masse des motifs répétitifs dérivés du composé siloxane est de 10 % en masse ou plus à 80 % en masse ou moins, par rapport à la masse du copolymère.

12. Copolymère selon la revendication 10 ou 11, dans lequel au moins l'un des autres monomères polymérisables est un monomère (méth)acrylique autre qu'un composé siloxane, et une proportion massique totale des motifs répétitifs dérivés du composé siloxane et des motifs répétitifs dérivés du monomère (méth)acrylique est de 50 % en masse ou plus, par rapport à la masse du copolymère.

13. Hydrogel comprenant le polymère selon la revendication 9 ou le copolymère selon l'une quelconque des revendications 10 à 12.

14. Matériau médical comprenant le polymère selon la revendication 9 ou le copolymère selon l'une quelconque des revendications 10 à 12.

15. Procédé de préparation du composé siloxane représenté par la formule (1) suivante : 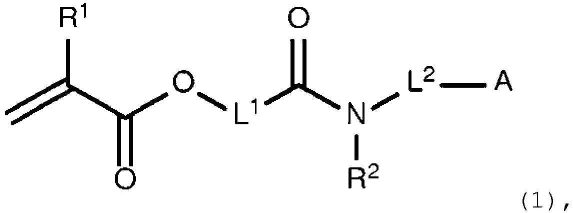
dans laquelle R¹ est un atome d'hydrogène ou un groupe méthyle, R² est un atome d'hydrogène ou un groupe hydrocarboné monovalent ayant de 1 à 6 atomes de carbone, L¹ est un groupe hydrocarboné divalent ayant de 1 à 5 atomes de carbone, L² est un groupe hydrocarboné divalent ayant de 2 à 10 atomes de carbone et pouvant contenir une liaison éther, et A est un motif polysiloxane représenté par la formule suivante (2) ou (3) : 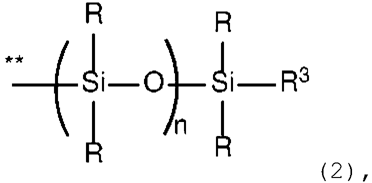 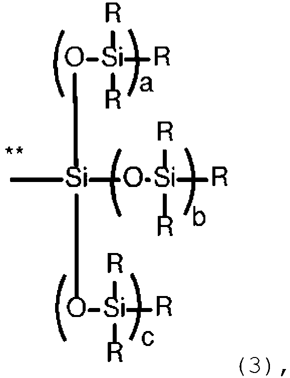
dans laquelle n est un entier de 1 à 100, a est un entier de 0 à 10, b est un entier de 0 à 10, et c est un entier de 0 à 10, à condition que a + b + c soit 2 ou plus, R et R³ sont, indépendamment l'un de l'autre, un groupe hydrocarboné monovalent ayant 1 à 10 atomes de carbone, et le site marqué par ** est une position de liaison au groupe L² dans la formule (1),
dans lequel le procédé comprend une étape de mise en réaction d'un composé siloxane possédant un groupe amino et représenté par la formule (5) suivante : 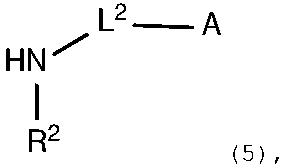
dans laquelle R², L² et A sont tels que décrits ci-dessus, avec un composé lactone ayant de 2 à 6 atomes de carbone pour obtenir un composé siloxane représenté par la formule (4) suivante : 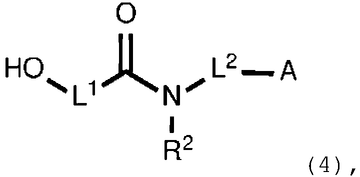
dans laquelle R², L¹, L² et A sont tels que décrits ci-dessus,
et une étape de mise en réaction du composé siloxane représenté par ladite formule (4) avec un halogénure d'acide (méth)acrylique pour obtenir le composé représenté par ladite formule (1).

16. Procédé selon la revendication 15, dans lequel le composé lactone est choisi dans le groupe constitué de la α-acétolactone, de la β-propiolactone, de la γ-butyrolactone, de la γ-valérolactone, de la δ-valérolactone et de la ε-caprolactone.

17. Procédé selon la revendication 15 ou 16, dans lequel l'halogénure d'acide (méth)acrylique est le chlorure d'acide méthacrylique, le chlorure d'acide acrylique, le bromure d'acide méthacrylique ou le bromure d'acide acrylique.

18. Procédé selon l'une quelconque des revendications 15 à 17, dans lequel une proportion du composé siloxane ayant une structure spécifique parmi les structures représentées par ladite formule (1) est de 95 % en masse ou plus.
